Europäisches Patentamt

European Patent Office

Office européen des brevets

(11) Publication number:　· **0 104 044**
**B1**

(12) **EUROPEAN PATENT SPECIFICATION**

(45) Date of publication of patent specification: **31.05.89**

(21) Application number: **83305370.5**

(22) Date of filing: **14.09.83**

(51) Int. Cl.⁴: **A 61 B 17/60**

(54) **External fixation system for bone fractures.**

(30) Priority: **16.09.82 US 418705**

(43) Date of publication of application:
**28.03.84 Bulletin 84/13** .

(45) Publication of the grant of the patent:
**31.05.89 Bulletin 89/22**

(84) Designated Contracting States:
**AT CH DE FR GB LI**

(56) References cited:
**CH-A- 203 544**
**DE-A-2 825 481**
**FR-A- 563 177**
**FR-A-2 405 063**
**GB-A- 29 174**
**GB-A- 483 903**
**GB-A-1 374 511**
**GB-A-1 480 167**
**GB-A-2 031 508**
**GB-A-2 040 168**
**GB-A-2 104 782**
**US-A-4 456 004**

(73) Proprietor: **Edwards, Charles Cannon**
**3907 Greenway**
**Baltimore Maryland 21218 (US)**

(72) Inventor: **Edwards, Charles Cannon**
**3907 Greenway**
**Baltimore Maryland 21218 (US)**

(74) Representative: **Moore, James William, Dr. et al**
**Pfizer Limited Ramsgate Road**
**Sandwich Kent CT13 9NJ (GB)**

Courier Press, Leamington Spa, England.

## Description

External fixation systems have been known for many decades. Such systems generally include a framework mounted around the bone fracture with a series of pins extending from the framework into the bone. Various forms of such systems are known including a quadrilateral system conventionally practiced with a Vidal frame as well as bilateral systems and unilateral systems. In general these systems promote bone union by stabilizing the position of the bone fragments. For some fracture patterns it is possible to compress the bone segments so as to achieve greater stability and speed union. However, in fractures with extensive comminution or bone loss, the fracture cannot be compressed without limit or shortening will occur.

In exemplary practice, such frames are used for treating various leg bone fractures. When using rigid external fixation frames, the majority of force from muscle pull or ambulation is transmitted through the frame and not the bone. After the healing process has begun, however, with the formation of soft callus between the bone ends, it would be desirable to permit the patient to exert a limited load on the bone and callus so as to encourage a stronger union. Conventional systems, however, either apply one static compression or static neutralization.

GB 1374511 discloses an external fixation system wherein sets of pins are mounted from a frame on longitudinally opposite sides of a bone fracture, said frame including a coupling comprising a rod for being generally longitudinally disposed from one set of pins on one side of the fracture to another set of pins on the other side of the fracture, a sleeve member having a longitudinal passageway extending therethrough, said rod being slidably positioned in said sleeve member, remote stop means coupled to said rod on the side of said sleeve member remote from the fracture, inner stop means coupled to said rod on the side of said sleeve member toward the fracture, and adjustable locking means for selectively locking said inner stop means to said rod at a preselected position thereon to control the distance between said stop means, whereby the degree of sliding movement of said rod within said sleeve member may be varied to provide a dynamic coupling thereof.

An object of the present invention is to provide improvements in external fixation systems which permit the selective application of axial force across the fracture site while maintaining the desired level of baseline compression, if any.

A further object of the present invention is to provide such a system which includes a dynamic coupling that can selectively maintain the frame in static neutralization or permit a relative sliding movement of a frame rod so that force could be applied across the fracture site while protecting the bone and fracture against excessive angulation or translation.

Thus according to the present invention there is provided an external fixation system wherein sets of pins are mounted from a frame on longitudinally opposite sides of a bone fracture, said frame includes a coupling comprising a rod for being generally longitudinally disposed from one set of pins on one side of the fracture to another set of pins on the other side of the fracture, a sleeve member having a longitudinal passageway extending therethrough, an end of said rod being slidably positioned in said sleeve member, remote stop means coupled to said rod on the side of said sleeve member remote from the fracture, inner stop means coupled to said rod on the side of said sleeve member toward the fracture, and adjustable locking means for selectively locking said inner stop means to said rod at a preselected position thereon to control the distance between said stop means whereby the degree of sliding movement of said rod within said sleeve member may be varied to provide a dynamic coupling thereof, characterized in that said sleeve member comprises a housing having an outer bore and a stabilizing insert and an inner sleeve, said inner sleeve slidably receiving said rod, with the outer diameter of said inner sleeve being smaller than the diameter of said outer bore, the stabilizing insert being adapted to be positioned within said housing to prevent tilting of said inner sleeve within said outer bore and said housing being adapted to be disassembled to permit removal of said insert to permit tilting of said inner sleeve within said outer bore.

Thus the external fixation system includes as at least one of the frame components a dynamic coupling. The dynamic coupling comprises a sleeve member and a pair of stop members all of which would be mounted to a respective rod. The stop member which is located closer to the bone fracture could be adjustably positioned at various locations on its rod. When both stop members simultaneously abut the sleeve member, the frame is in static neutralization and will absorb any load applied thereto rather than permitting the load to bear directly against the bone fracture. When, however, the inner stop member is adjusted to a position out of contact with the sleeve member, a clearance is provided which would permit the rod to slide in the sleeve member a distance equal to the distance between the stop members. Under these conditions, a load would be applied directly to the bone fracture or healing callus.

The outer stop makes it possible to compress certain fractures with adjustable rods while allowing zero to unlimited impact load to cross the fracture site depending on the position of the inner stop.

One or both stop members may include cushioning means or shock absorbing members such as elastomeric washers disposed for contacting the sleeve member as the rod slides back and forth.

One or both stop members may be in the form of a split sleeve mounted around the rod with adjustable clamp means spanning the open ends of the split sleeve so as to selectively permit the locked position of the stop member on the rod to be varied.

Figure 1 is a side elevation view showing one form of an external fixation system which incorporates the invention;

Figure 2 is a front elevation view partly in section of a portion of the system shown in Figure 1;

Figure 3 is a top plan view partly in section of the portion of the system shown in Figure 2;

Figure 4 is a rear elevation view partly in section of the portion of the system shown in Figures 2—3;

Figures 5 and 6 are cross-sectional views taken through Figure 2 along the lines 5—5 and 6—6;

Figure 7 is a side elevation view of an alternative external fixation system which also incorporates the invention;

Figures 8—10 are elevation views showing a portion of the system in different phases of operation; and

Figures 11 and 12 are perspective views schematically illustrating the use of the invention in various frame arrangements.

In general the invention includes the incorporation of a novel dynamic coupling as at least one of the frame components in an external fixation system. In the late 1930's Dr. Raoul Hoffman designed an external fixation system. Such systems have been known in the art utilizing various frame forms. The invention will be described with particular reference to the Vidal frame which is a quadrilateral frame. It is to be understood, however, that such description is merely exemplary and that the invention may also be practiced with other forms of quadrilateral frames and indeed with other types of external fixation systems including bilateral and unilateral frames. Since the details of conventional external fixation systems are known, it is not necessary to repeat a description herein except as would facilitate an understanding of the invention. A description of an external fixation system of the type illustrated and the conventional components therein is found in a publication entitled "The Original Hoffman External Fixation System" copyright 1979 by Howmedica, Inc., the details of which are incorporated herein by reference thereto.

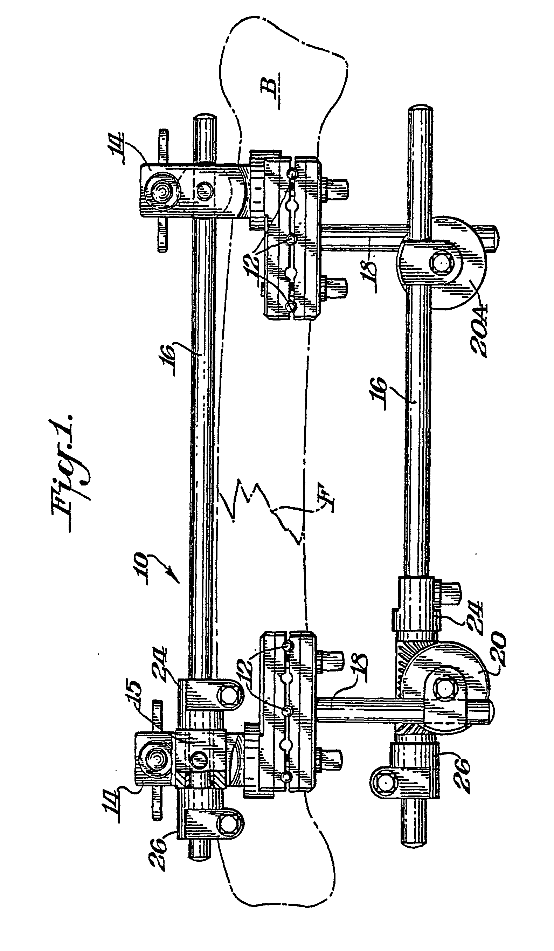

Figure 1 illustrates an external fixation system 10 of the type conventionally known as a Vidal frame but which includes a number of dynamic couplings. As illustrated therein, system 10 is used for treating a fracture F. System 10 includes sets of transfixing pins 12 which are secured through the bone B on each side of fracture F. The transfixing pins 12 are then mounted to universal ball joints 14. In the known Vidal system, adjustable connecting rods would longitudinally span each pair of universal ball joints. In accordance with the practice of the invention, however, a more simplified form of rod 16 may be utilized. As illustrated in Figure 1, rods 16 are simply straight members having the same diameter as the rods conventionally used in the known Vidal frame. Ball joints 14 also include outwardly extending rods 18 which are received in couplings.

The frame may be modified by having the dynamic couplings 20 on only one side of the bone. In this variation the ball joint, such as parts B-10 or B-11 of the aforenoted publication, would be modified by replacing the rod gripping portion with a novel housing insert having a pair of nipples for fitting in the ball joint. The housing insert would have a bearing surface that would permit its rod to readily slide in accordance with the sliding permitted by couplings 20. The rod in the modified ball joint is preferably provided with stops, such as stops 26, at its outer ends.

Ordinarily with conventional Vidal frames, the couplings would be articulation couplings which would be locked to the longitudinal rods in such a manner that relative movement in the longitudinal direction between the rods and the couplings would be prevented. The conventional Vidal frame which includes adjustable connecting rods and conventional articulation couplings is capable of providing a compressive force to the bone or of maintaining the bone in a static neutralization condition. The rigid frame would absorb any load or weight that would have otherwise been transmitted to the bone.

While such a conventional frame is suitable, particularly in the initial stages of healing, it is desirable in later stages of healing to permit the patient to apply some load to the bone so as to stimulate a dynamic response in the bone since the bones are not static material but rather are viable and regenerate, which makes the bones biodynamic. The invention replaces the conventional articulation couplings with dynamic couplings 20 constructed in such a manner as to function in either a static neutralization condition or dynamically by permitting the transmission of loads through the fracture site in addition to any desired level of baseline compression.

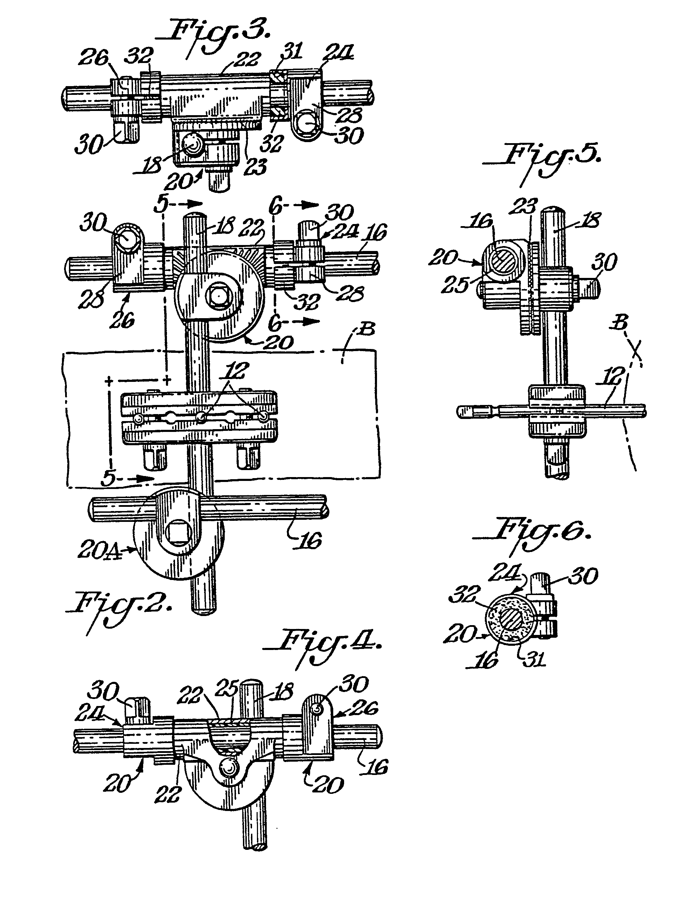

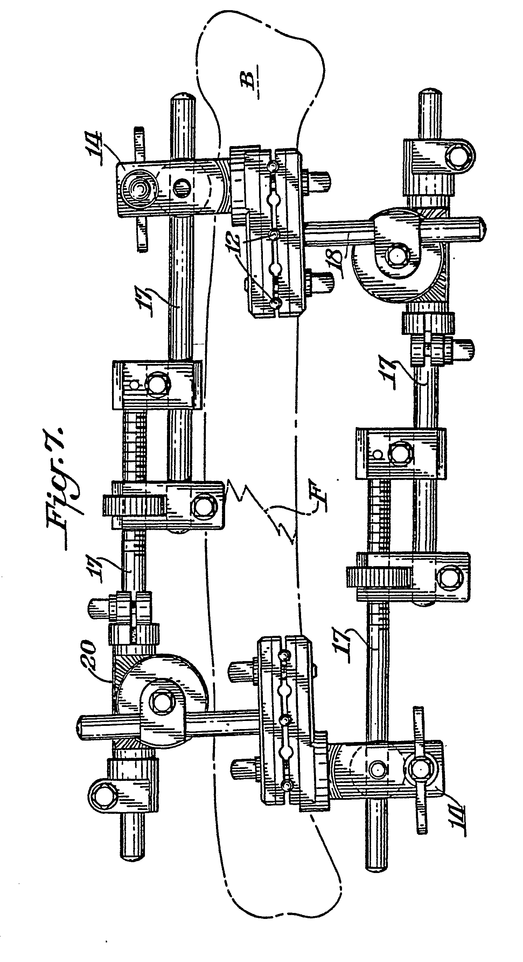

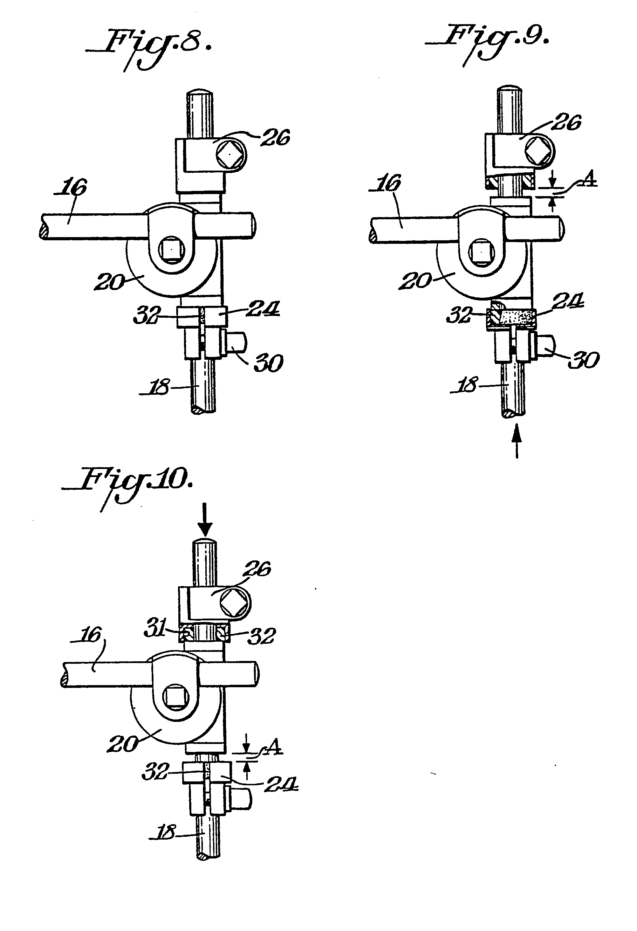

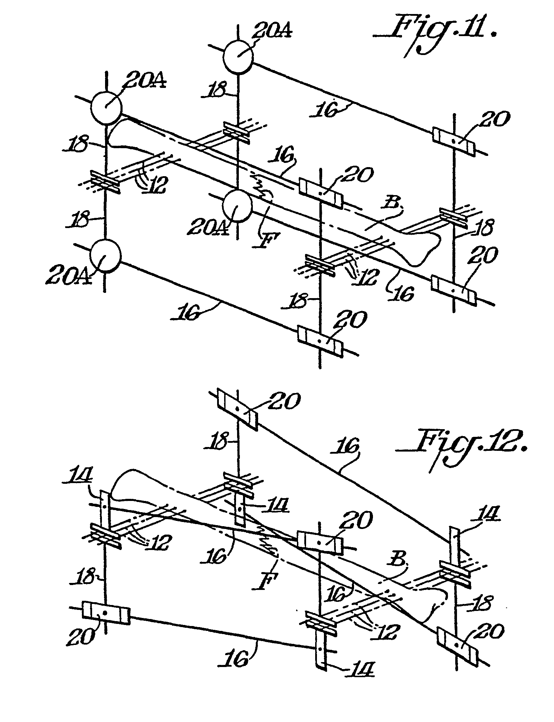

Figures 2—6 show the details of dynamic coupling 20. As indicated therein, coupling 20 essentially comprises a central sleeve member 22 with a stop member 24, 26 on each side thereof. Figure 5 illustrates sleeve member 22 to include ratchet formations 23 for providing angular adjustment of rod 16. As illustrated in Figure 5, sleeve member 22 includes structure to the right hand side of ratchet formations 23 for receiving depending rod 18. The structure to the right hand or inner side of ratchet formations 23 can be generally the same as corresponding structure in conventional articulation couplings. The structure to the outward side of ratchet formations 23, however, is novel and is so designed as to permit a dynamic coupling to result.

The ratchet formations 23 could be replaced with homogeneously roughened facing surfaces prepared by casting or other suitable methods. This would permit a virtually infinite number of gradations in the relative angular position between rods 16 and 18, thereby making parallelity of the longitudinal rods 16 easier to achieve, thus reducing friction against axial sliding.

As illustrated in Figure 4, sleeve member 22 includes a liner 25 made of a material such as Teflon® to facilitate a sliding movement of rod 16 within the sleeve member 22.

Stop member 26 is located toward the outer end of rod 16 and may be referred to as a remote stop member in that it would be remote from the fracture. Stop member 26 functions primarily to provide an outer limit for the relative path of travel of rod 16 through sleeve member 22. This makes it possible to apply compression across the fracture site either manually or with adjustable rods. Stop member 26 is illustrated in a form that permits its location on rod 16 to be varied. Once locked in place, however, it would remain stationary during the healing process. The adjustability feature of stop member 26 is also desirable for convenience of assembling the components of coupling 20. From a concept standpoint, however, stop member 26 need not be adjustably mounted and could take any suitable form such as being a cap friction fitted over the end of rod 16. In the illustrated form, however, stop member 26 is a split sleeve having a pair of offset flanges 28 at the open ends thereof with a suitable fastener 30 such as a threaded fastener serving as a clamp to lock stop member 26 in place at a desired location on rod 16.

Stop member 24 is of similar construction to remote stop member 26. Stop member 24 may be considered as an inner stop member in that it is located closer to the bone fracture. Inner stop member 24 serves an important function in permitting and controlling the amount of back and forth movement of rod 16 in sleeve member 22. Accordingly, inner stop member 24 should be adjustably locked to rod 16 in such a manner that its position can be changed when desired.

Another desirable feature is the inclusion of cushioning members or shock absorbing means in each stop member located in the path of relative movement of the sleeve member 22 with respect to the moving rod 16. In the preferred form, the shock absorbing means is an elastomeric washer 32 which absorbs energy of impact upon axial loading and dampens the noise which would otherwise result upon contact of metal to metal when sleeve member 22 is contacted by each stop member. Any suitable cushioning or shock absorbing material could be used. The material, however, should be an autoclavable high temperature and high performance material. A material commercially known under the 3M trademark Fluorel® may be satisfactorily used.

If it is desired to initially apply a compressive force to the bone, a conventional external fixation system such as a Vidal frame could be used, with either standard or dynamic couplings 20. Alternatively at a suitable time during the healing process the articulation couplings would be sequentially replaced by the dynamic couplings 20. Where, for example, a quadrilateral frame is used, the sequential replacements could be made without affecting the static condition of the bone. During this early stage of healing, the components of coupling 20 would be in the position indicated in Figure 8 where each of the stop members 24 and 26 is juxtaposed sleeve member 22. While in this position the frame is in the static condition which would prevent a load being applied or transmitted through the fracture site. When it is determined that partial physiological loading would be desired to stimulate healing, locking member 30 on inner adjustable stop 24 would be manipulated to loosen inner stop 24 and inner stop 24 would be moved away from sleeve member 22. During this initial stage for stimulating dynamic response, the distance "A" that stop member 24 would be moved might be about 1—2 mm. The gap "A" would thus permit relative sliding movement of rod 16 in sleeve member 22 by the distance of that gap. As the healing process continues and the deformable callus strengthens, it may be desirable to permit greater movement. In this case the position of stop member 24 would be adjusted to provide an even greater gap. As is apparent from Figures 9 and 10, stop members 24, 26 define the ends of the path of movement. Thus, for example, coupling 20 at one extreme condition would have remote stop member 26 abutting against sleeve member 22. In the other extreme condition, stop member 24 would abut against sleeve member 22.

In order to alter the frame in such a way as to permit more fracture flexibility and facilitate the latter stages of fracture healing, the set screws 30 on either the standard or dynamic couplings can be loosened. Screws 30 are flanged or otherwise modified to prevent their dislodgement after loosening. Loosening the screws has two benefits in the latter stages of fracture healing: (1) to provide greater freedom of motion in all planes; and (2) to facilitate axial translation (fracture site loading) by reducing friction against sliding in cases where the longitudinal rods are not parallel.

A particular advantage of the dynamic couplings is that the very same elements may be used for both static neutralization and various gradations of a dynamic condition by merely making slight manipulations rather than requiring major manipulations or complete substitutions of components.

The dynamic coupling would include the conventional coupling components so that the coupling may also be selectively adjusted to vary the orientation of the rod 16 or 17 about its own axis and about an axis perpendicular to its own axis as well as linearly in a vertical plane which are the adjustments achievable with conventional articulation couplings.

In the illustrated system where the invention is used with fractures of large bones, a 8 mm rod 16 would be used for sliding in sleeve member 22 of generally the same diameter. Rod 16 would be available in several lengths in accordance with the desired application. To facilitate the sliding action where a plurality of rods 16 and dynamic couplings are used, it is desired to have the rods 16 as parallel as possible. However, the later described double bore design may be used in cases where the rods cannot be made relatively parallel. As previously indicated, each stop member preferably has a washer 32 affixed in a respective recess 31 of its stop member to eliminate clicking noise during fracture unloading during ambulation, to buffer against excessive compression across the fracture and fixateur and to absorb impact loads. The washer should have medium elasticity such as 50 durometers and might be several millimeters in thickness. Stop member 24, 26 as well as sleeve member 22 may be made from stainless steel with set screws 30 functioning to lock each stop member in place. Where, however, other size bones or different applications are being used, the dimensions of the components and their materials might differ in accordance with the desired results.

As previously indicated, the specific description herein of the dynamic couplings being incorporated in a specific form of Vidal frame is not intended to be limiting. Figures 11 and 12, for example, show other variations where the dynamic couplings 20 are located differently. These variations also include the conventional articulation couplings indicated by the reference numeral 20A where appropriate. It is also noted that the same reference numerals are used for the various universal ball joints and adjustable connecting rods although various frame arrangements may require the utilization of different variations of those components.

As also previously noted, the invention is not limited to the practice of a Vidal frame. Rather the invention can be used with any form of external fixation system with appropriate modification.

The present invention which includes dynamic couplings thus allows the transmission of controlled axial loading to a fracture stabilized by an external fixation system while simultaneously maintaining a preset minimum compressive force if desired (see Figure 7) and preventing significant angular or torsional displacements across the fracture. The invention enables application of increased axial load to a fracture by means of a dynamic coupling. In the preferred practice of the invention, a sliding rod/coupling articulation system is used. The invention may be practiced by interposing a deformable material between the rod and coupling and by providing a sliding joint within the coupling which is attached to a standard free rod or the rod portion of an adjustable rod. Advantageously the invention allows physiological axial loading of a healing fracture by use of components which are compatible with standard Hoffman external fixation system components.

As previously described, the invention encompasses various features to overcome the increased friction against sliding which results from minor non-parallelity of the longitudinal rods (e.g. 16 or 17). In certain complex cases, however, major divergence of the longitudinal rods cannot be avoided. In this circumstance a double bore dynamic coupling (DBDC) is used. The DBDC compensates for rod divergence by allowing an inner sleeve to tilt within an outer housing.

The DBDC consists of a housing, an inner sleeve, and a stabilizing insert. When assembled the housing, inner sleeve and insert together function the same as the previously described sleeve member 22. When the insert is removed, however, the inner sleeve can tilt within the bore of the housing. The inner diameter of the housing is 1—4 mm greater than the outer diameter of the inner sleeve. Flanges on each end of the inner sleeve extend beyond the ends of the housing to keep the inner sleeve within the housing. In its preferred embodiment the inner sleeve is lined with Teflon® or other appropriate bearing material.

The housing is split diametrically in order to place or remove the stabilizing insert which wedges between the housing and inner sleeve. The bore within the housing is reconstituted by assembling the two pieces of the housing by means of interlocking edges on one side of the housing and the coupling set screw on the other.

One refinement is the provision of a plurality of stabilizing inserts of varying thicknesses. By replacing thicker inserts with thinner inserts as healing progresses, greater freedom of motion and efficiency of sliding are promoted.

The dynamic coupling and the various other features of this invention should make it possible for the healing physician to better match the mechanical characteristics of the external fixation device with the biologic needs of the fracture.

## Claims

1. An external fixation system wherein sets of pins are mounted from a frame on longitudinally opposite sides of a bone fracture,
said frame includes a coupling (20) comprising a rod (16) for being generally longitudinally disposed from one set of pins (12) on one side of the fracture to another set of pins (12) on the other side of the fracture, a sleeve member (22) having a longitudinal passageway extending therethrough, an end of said rod (16) being slidably positioned in said sleeve member (22), remote stop means (26) coupled to said rod (16) on the side of said sleeve member (22) remote from the fracture, inner stop member (24) coupled to said rod (16) on the side of said sleeve member (22) toward the fracture, and adjustable locking means (30) for selectively locking said inner stop means (24) to said rod (16) at a preselected position thereon to control the distance between said stop means (24, 26), whereby the degree of sliding movement of said rod (16) within said sleeve member (22) may be varied to provide a dynamic coupling thereof, characterized in that said sleeve member (22) comprises a housing having an outer bore and a stabilizing insert and an inner sleeve, said inner sleeve slidably receiving said rod (16), with the outer diameter of said inner sleeve being smaller than the diameter of said outer bore, the stabilizing insert being adapted to be positioned within said housing to prevent tilting of said inner sleeve within said outer bore and said housing being adapted to be disassembled to permit removal of said insert to permit tilting of said inner sleeve within said outer bore.

2. The system of claim 1, further characterized in that said coupling (20) further includes shock absorber means (32) in at least one of said stop means (24, 26) disposed toward said sleeve member (22).

3. The system of claim 2, further characterized in that said coupling (20) includes shock absorber means (32) in each of said stop means (24, 26).

4. The system of claim 2, further characterized in that said shock absorber means is a washer (32) mounted in its stop means (24, 26) around said rod (16).

5. The system of claim 4, further characterized in that said washer (32) is made from an elastomeric material.

6. The system of claim 1, further characterized in that said inner stop means (24) is a split sleeve mounted around said rod (16), and said adjustable locking means comprises a clamp means (30) spanning the open ends of said split sleeve.

7. The system of claim 1, further characterized in that said coupling (20) further includes means (20A) for selectively adjusting the orientation of said rod (16) about its own axis and about an axis perpendicular to its own axis and linearly in a plane perpendicular to said pins.

8. The system of claim 1, further characterized in that said system includes a plurality of said couplings (20).

9. The system of claim 8, further characterized in that said frame is a quadrilateral frame and said coupling (20) is mounted at selected corners of said frame.

10. The system of claim 9, further characterized in that at least one of said rods (16) is a single straight element spanning a respective pair of said sets of pins (12).

**Patentansprüche**

1. Äußerliche Befestigungsvorrichtung, bei der Sätze von Stiften an in Längsrichtungen gegenüberliegenden Seiten eines Knochenbruchs von einem Rahmen gehalten sind, wobei der Rahmen eine Verbindung (20) umfaßt, die eine Stange (16), die im allgemeinen in Längsrichtung von einem Satz Stifte (12) auf der einen Seite des Bruches zu einem anderen Satz Stifte (12) auf der anderen Seite des Bruches angeordnet werden kann, ein Hülsenelement (22) mit einem längs hindurch sich erstreckenden Durchgang, wobei ein Ende der Stange (16) in dem Hülsenelement (22) gleitbar vorgesehen ist, mit der Stange (16) an der von dem Bruch entfernten Seite des Hülsenelementes (22) verbundene äußere Anschlagmittel (26), mit der Stange (16) an der dem Bruch zugewandten Seite des Hülsenelementes verbundene innere Anschlagmittel (4) und einstellbare Sperreinrichtungen (30) zum wahlweisen Sperren der inneren Anschlagmittel (24) auf der Stange (16) in einer vorher ausgewählten Stellung, um den Abstand zwischen den Anschlagmitteln (24, 26) zu regeln, wodurch das Maß der Gleitbewegung der Stange (16) in dem Hülsenelement (22) variiert werden kann, um eine dynamische Verbindung zu erhalten, aufweist, dadurch gekennzeichnet, daß das Hülsenelement (22) ein Gehäuse mit einer äußeren Bohrung und einem stabilisierendem Einsatzstück und einer inneren Hülse umfaßt, die innere Hülse die Stange (16) gleitbar aufnimmt, wobei der Außendurchmesser der inneren Hülse kleiner is als der Durchmesser der äußeren Bohrung, das stabilisierende Einsatzstück geeignet ist, innerhalb des Gehäuses angeordnet zu werden, um ein Kippen der inneren Hülse innerhalb der äußeren Bohrung zu verhindern, und das Gehäuse geeignet ist, auseinandergenommen zu werden, um das Entfernen des Einsatzstückes zu erlauben, um das Kippen der inneren Hülse innerhalb der äußeren Bohrung zu erlauben.

2. Vorrichtung nach Anspruch 1, dadurch gekennzeichnet, daß die Verbindung (20) in mindestens einem der Anschlagmittel (24, 26) weiters Stoßdämpfer (32) aufweist, die dem Hülsenelement (22) zugewandt angeordnet sind.

3. Vorrichtung nach Anspruch 2, dadurch gekennzeichnet, daß die Verbindung (20) in jedem der Anschlagmittel (24, 26) Stoßdämpfer (32) aufweist.

4. Vorrichtung nach Anspruch 2, dadurch gekennzeichnet, daß die Stoßdämpfer eine Beilagscheibe (32) sind, die in den Anschlagmitteln (24, 26) um die Stange (16) herum angeordnet ist.

5. Vorrichtung nach Anspruch 4, dadurch gekennzeichnet, daß die Beilagscheibe (32) aus einem elastomeren Material hergestellt ist.

6. Vorrichtung nach Anspruch 1, dadurch gekennzeichnet, daß die inneren Anschlagmittel (24) eine Schlitzhülse sind, die um die Stange (16) angeordnet ist, und die einstellbaren Sperreinrichtungen eine Klemmeinrichtung (30) umfassen, die die offenen Enden der Schlitzhülse zusammenspannt.

7. Vorrichtung nach Anspruch 1, dadurch gekennzeichnet, daß die Verbindung (20) weiters Einrichtungen (20A) zur wahlweisen Einstellung der Orientierung der Stange (16) um ihre eigene Achse und um eine Achse senkrecht zur eigenen Achse und linear in einer Ebene senkrecht zu den Stiften aufweist.

8. Vorrichtung nach Anspruch 1, dadurch gekennzeichnet, daß sie eine Mehrzahl von Verbindungen (20) umfaßt.

9. Vorrichtung nach Anspruch 8, dadurch gekennzeichnet, daß der Rahmen ein vierseitiger Rahmen ist und die Verbindung (29) an ausgewählten Ecken des Rahmens angeordnet ist.

10. Vorrichtung nach Anspruch 9, dadurch gekennzeichnet, daß mindestens eine der Stangen (16) ein einziges gerades Element ist, das ein entsprechendes Paar von Sätzen von Stiften (12) überspannt.

**Revendications**

1. Système de fixation externe selon lequel des jeux de broches sont montés à partir d'un bâti sur les faces longitudinalement opposées d'une fracture osseuse,

ledit bâti comprenant un accouplement (20) formé d'une tige (16) adaptée à être disposée d'une manière générale longitudinalement à partir d'un jeu de broches (12) sur un côté de la fracture jusqu'à un autre jeu de broches (12), sur l'autre côté de la fracture, un élément formant manchon (22) ayant un passage longitudinal le traversant, une extrémité de ladite tige (16) étant positionnée de façon coulissante dans ledit élément formant manchon (22), des moyens d'arrêt éloignés (26) accouplés à ladite tige (16) sur la face dudit élément formant manchon (22) éloignée de la fracture, des moyens d'arrêt internes (24) accouplés à ladite tige (16) sur le côté de l'élément formant manchon (22) en direction de la fracture, des moyens de verrouillage réglables (30) pour verrouiller sélectivement lesdits moyens d'arrêt internes (24) sur ladite tige (16) en une position présélectionnée de celle-ci pour régler la distance entre lesdits moyens d'arrêt (24, 26), grâce à quoi le degré du mouvement de coulissement de ladite tige (16) dans ledit élément formant manchon (22) peut être varié pour offrir un accouplement dynamique entre ceux-ci, caractérisé en ce que ledit élément formant manchon (22) comprend un boîtier ayant un perçage extérieur et une pièce rapportée de stabilisation, et un manchon interne, ledit manchon interne recevant de manière coulissante ladite tige (16), le diamètre externe dudit manchon interne étant plus petit que le diamètre dudit perçage externe, la pièce rapportée de stabilisation étant adaptée à être disposée dans ledit boîtier pour empécher l'inclinaison dudit manchon interne à l'intérieur dudit perçage externe et ledit boîtier étant adapté à être démonté pour permettre l'enlèvement de ladite pièce rapportée et l'inclinaison dudit manchon interne à l'intérieur dudit perçage externe.

2. Système selon la revendication 1, caractérisé en outre en ce que ledit accouplement (20) comprend de plus des moyens absorbant les chocs (32) dans l'un au moins desdits éléments d'arrêt (24, 26) disposés en direction dudit élément formant manchon (22).

3. Système selon la revendication 2, caractérisé en outre en ce que ledit accouplement (20) comprend des moyens absorbant les chocs (32) dans chacun desdits moyens d'arrêt (24, 26).

4. Système selon la revendication 2, caractérisé en outre en ce que lesdits moyens absorbant les chocs sont constitués par une rondelle (32) montée dans le moyen d'arrêt correspondant (24, 26) autour de ladite tige (16).

5. Système selon la revendication 4, caractérisé en outre en ce que ladite rondelle (32) est faite en un matériau élastomère.

6. Système selon la revendication 1, caractérisé en outre en ce que ledit élément d'arrêt interne (24) est un manchon fendu monté autour de ladite tige (16), et en ce que lesdits moyens de verrouillage réglables sont constitués d'un moyen formant pince (30) allant d'un côté à l'autre des extrémités ouvertes dudit manchon fendu.

7. Système selon la revendication 1, caractérisé en outre en ce que ledit accouplement (20) comprend de plus des moyens (20a) pour régler sélectivement l'orientation de ladite tige (16) autour de son propre axe et autour d'un axe perpendiculaire à son propre axe et linéairement dans un plan perpendiculaire auxdites broches.

8. Système selon la revendication 1, caractérisé en outre en ce que ledit système comprend une pluralité desdits accouplements (20).

9. Système selon la revendication 8, caractérisé en outre en ce que ledit bâti est un bâti en quadrilatère et en ce que ledit accouplement (20) est monté à des angles sélectionnés dudit bâti.

10. Système selon la revendication 9, caractérisé en outre en ce que l'une au moins desdits tiges (16) est un élément rectiligne unique allant d'un côté à l'autre d'une paire respective desdites jeux de broches (12).

# Fig.1.

*Fig.3.*

*Fig.5.*

*Fig.2.*

*Fig.6.*

*Fig.4.*

Fig. 7.

EP 0 104 044 B1

*Fig.8.*

*Fig.9.*

*Fig.10.*

4

Fig.11.

Fig.12.

5